(19)

**Europäisches Patentamt**
**European Patent Office**
**Office européen des brevets**

(11) **EP 4 555 926 A1**

(12) **EUROPEAN PATENT APPLICATION**
published in accordance with Art. 153(4) EPC

(43) Date of publication:
**21.05.2025 Bulletin 2025/21**

(21) Application number: **22956420.8**

(22) Date of filing: **23.08.2022**

(51) International Patent Classification (IPC):
**A61B 5/11** (2006.01)   **A61B 5/01** (2006.01)
**A61B 5/113** (2006.01)

(52) Cooperative Patent Classification (CPC):
**A61B 5/01; A61B 5/11; A61B 5/113**

(86) International application number:
**PCT/JP2022/031638**

(87) International publication number:
**WO 2024/042592 (29.02.2024 Gazette 2024/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **MITSUBISHI ELECTRIC
CORPORATION
Chiyoda-ku
Tokyo 100-8310 (JP)**

(72) Inventors:
• **KITAMURA, Takayuki
Tokyo 100-8310 (JP)**
• **KAGEME, Satoshi
Tokyo 100-8310 (JP)**
• **KOBAYASHI, Satoi
Tokyo 100-8310 (JP)**

• **YOSHIKI, Wataru
Tokyo 100-8310 (JP)**
• **SAKAMOTO, Katsumasa
Tokyo 100-8310 (JP)**
• **MATSUBARA, Tsutomu
Tokyo 100-8310 (JP)**
• **YAMAMOTO, Tomoyuki
Tokyo 100-8310 (JP)**
• **KOMORI, Takuya
Tokyo 100-8310 (JP)**
• **ISOBE, Yuma
Tokyo 100-8310 (JP)**
• **SUZUKI, Namihei
Tokyo 100-8310 (JP)**

(74) Representative: **Pfenning, Meinig & Partner mbB
Patent- und Rechtsanwälte
An der Frauenkirche 20
01067 Dresden (DE)**

(54) **VITAL MEASUREMENT DEVICE AND VITAL MEASUREMENT METHOD**

(57)   A vital measuring device including: a single infrared sensor (1) that captures an infrared image of a target object, recognizes a living body from the captured infrared image, and estimates a body temperature and a two-dimensional orientation of the recognized living body; a single radar sensor (2) temporally synchronized with the infrared sensor and disposed in proximity to the infrared sensor, the single radar sensor (2) having a signal acquisition unit (221) that acquires a reception signal of a reflected wave from an antenna that receives the reflected wave by the target object, a signal separation unit (222) that separates the acquired reception signal into a moving object signal and a stationary object signal, a moving object estimating unit (223) that detects a moving object by estimating a range, a velocity, and a two-dimensional orientation of the moving object from the moving object signal, and a radar vital estimating unit (224) that estimates a position, a respiration rate, and a heart rate of the living body from the stationary object signal on the basis of a detection result of the detected moving object; and a final vital output unit (3) to acquire and output the estimated position, respiration rate, heart rate, and body temperature of the living body.

EP 4 555 926 A1

FIG. 7

START

Infrared Sensor Estimates Body Temperature and Two-Dimensional Orientation of Living Body — ST1

Signal Acquisition Unit of Radar Sensor Acquires Reception Data — ST2

Signal Separation Unit of Radar Sensor Separates Moving Object Signal and Stationary Object Signal — ST3

Moving Object Estimating Unit of Radar Sensor Estimates Position of Moving Object — ST4

Does Stationary Determination Unit of Radar Sensor Determine that Living Body Detected by Infrared Sensor is Stationary? — ST5

NO

YES

Fourier Transform Unit of Radar Sensor Performs Fourier Transform on Stationary Object Signal in Range Direction — ST6

Digital Beam Forming Unit of Radar Sensor Performs Two-Dimensional Beam Forming on Stationary Object Signal in Biological Detection Orientation in Infrared Sensor — ST7

Presence Position Specifying Unit of Radar Sensor Estimates Three-Dimensional Position of Living Body — ST8

ST9 — Respiration Rate Estimating Unit of Radar Sensor Estimates Respiration Rate of Living Body

ST10 — Heart Rate Estimating Unit of Radar Sensor Estimates Heart Rate of Living Body

Final Vital Output Unit Outputs Body Temperature, Position, Respiration Rate, and Heart Rate of Living Body — ST11

END

**Description**

TECHNICAL FIELD

The present disclosure relates to a vital measuring technique.

BACKGROUND ART

There is a measuring system that combines a single infrared sensor and a plurality of radar sensors to measure vital signs of a plurality of persons to be measured (see Non-Patent Literature 1). Note that, in the present disclosure, a term "vital" is used as a term meaning the vital signs.

The infrared sensor of the measuring system includes a sensitivity sensor that receives infrared light emitted from a person to be measured and outputs a light reception signal. The infrared sensor can estimate the number and positions of the persons to be measured even under a low illuminance condition while considering privacy.

The radar sensor of the measuring system includes an antenna that transmits a microwave or a millimeter wave toward a person to be measured present in a room, receives a reflected wave that is the microwave or the millimeter wave after being reflected by the person to be measured, and outputs a reception signal of the reflected wave. In addition, the radar sensor includes a first mixer that extracts an in-phase signal included in the reception signal output from the antenna, a second mixer that extracts a quadrature signal included in the reception signal output from the antenna, and a signal processing device that generates a complex signal including the in-phase signal and the quadrature signal. Respiration rates and heart rates of the plurality of persons to be measured are estimated using the plurality of complex signals obtained from the plurality of radar sensors on the basis of an estimation result of the positions of the persons to be measured by the infrared sensor.

CITATION LIST

NON-PATENT LITERATURES

Non-Patent Literature 1: De-Ming Chian, Chao-Kai Wen, Chang-Jen Wang, Ming-Huan Hsu, and Fu-Kang Wang, "Vital Signs Identification System with Doppler Radars and Thermal Camera", IEEE Transactions on Biomedical Circuits and Systems, vol. 16, no. 1, pp. 153-167, Feb. 2022.

SUMMARY OF INVENTION

TECHNICAL PROBLEM

The measuring system disclosed in Non-Patent Literature 1 relates to measuring of vitals of a plurality of persons to be measured closely seated in a room. In order to detect the vitals of the plurality of closely spaced persons to be measured, it is necessary to use a plurality of radar sensors dispersedly arranged in the room, so that the measuring system is expensive. On the other hand, in watching over an elderly person in a service-equipped elderly residence or the like, in general, there are few cases of a plurality of persons whose vitals to be measured, and in many cases, it is sufficient if vitals of one elderly person can be constantly measured. In that case, there is a problem that the measuring system involves a high cost with respect to required performance.

The present disclosure has been made in view of the above problem, and an object of the present disclosure is to provide a vital measuring technique capable of detecting vitals of a person to be measured by using only a single infrared sensor and a single radar sensor.

SOLUTION TO PROBLEM

An aspect of a vital measuring device according to embodiments of the present disclosure includes: a single infrared sensor including a light receiving unit to receive infrared light emitted from a target object and capture an infrared image, a biological recognition unit to recognize a living body from the infrared image captured by the light receiving unit, and an infrared vital estimating unit to estimate a body temperature and a two-dimensional orientation of the living body recognized by the biological recognition unit; a single radar sensor temporally synchronized with the infrared sensor and disposed in proximity to the infrared sensor in such a manner as to sense the living body, the single radar sensor including a signal acquisition unit to acquire a reception signal of a reflected wave from an antenna for receiving the reflected wave from the target object, a signal separation unit to separate the reception signal acquired by the signal acquisition unit into a moving object signal and a stationary object signal, a moving object estimating unit to detect a moving object by estimating a range, a velocity, and a two-dimensional orientation of the moving object from the moving object signal, and a radar vital estimating unit to estimate a position, a respiration rate, and a heart rate of the living body from the stationary object signal on a basis of a detection result of the moving object detected by the moving object estimating unit; and a final vital output unit to acquire the position, the respiration rate, and the heart rate of the living body estimated by the radar vital estimating unit, acquire the body temperature of the living body estimated by the infrared vital estimating unit, to output the acquired position, body temperature, respiration rate, and heart rate of the living body.

ADVANTAGEOUS EFFECTS OF INVENTION

According to a vital measuring technique according to the embodiments of the present disclosure, it is possible to detect vitals of a person to be measured by using only a single infrared sensor and a single radar sensor.

BRIEF DESCRIPTION OF DRAWINGS

FIG. 1 is an overall configuration diagram illustrating a vital measuring device according to a first embodiment.
FIG. 2 is a hardware configuration diagram of an infrared sensor of the vital measuring device according to the first embodiment.
FIG. 3 is a hardware configuration diagram of a radar sensor of the vital measuring device according to the first embodiment.
FIG. 4 is a configuration diagram of a final vital output unit 3 of the vital measuring device according to the first embodiment.
FIG. 5 is a hardware configuration diagram illustrating hardware of the vital measuring device according to the first embodiment.
FIG. 6 is a hardware configuration diagram of a computer in a case where the vital measuring device is implemented by software, firmware, or the like.
FIG. 7 is a flowchart illustrating a vital measuring method which is a processing procedure performed by the vital measuring device.
FIG. 8 is an explanatory diagram illustrating up-chirp signals Tx(1) to Tx(N) with the number of hits Q generated by a signal generator 212a.
FIG. 9 is an explanatory diagram illustrating Fourier-transformed signals Ssta(r, g, h, c) of N pieces of stationary object reception data in a certain transmission cycle c.
FIG. 10 is an explanatory diagram illustrating beam forming signals BF(r, c) belonging to the same range bin r.
FIG. 11 is an explanatory diagram illustrating a change of a circle fitting error E(r) with respect to the range bin r.
FIG. 12 is an explanatory diagram illustrating a phase change signal $\theta(c)$ at a range bin r0.
FIG. 13 is an explanatory diagram illustrating vectors used for calculation of an inner product value corresponding to a certain adjacent beam forming signal point pair of the beam forming signals BF(r, c) belonging to the same range bin r.

DESCRIPTION OF EMBODIMENTS

Hereinafter, various embodiments of the present disclosure will be described in detail with reference to the accompanying drawings. Note that components denoted by the same or similar reference numerals in the drawings have the same or similar configurations or functions, and redundant description of such components will be omitted.

First Embodiment

<Configuration>

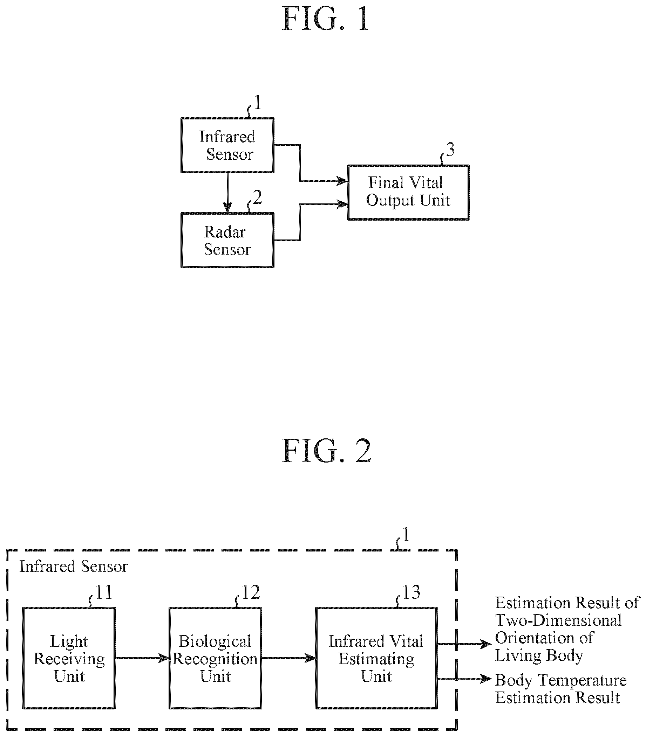

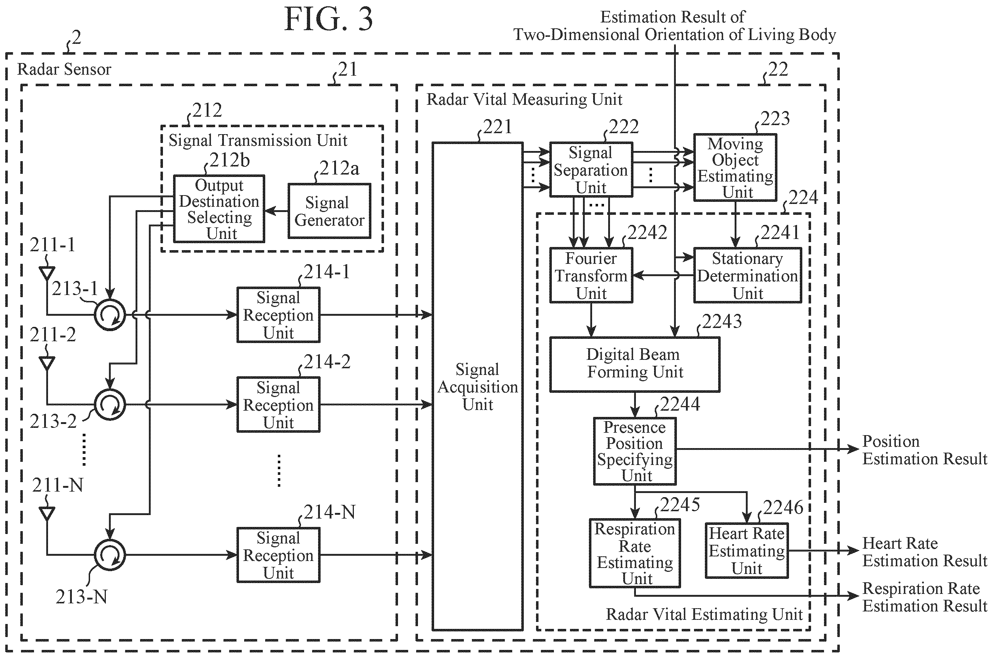

FIG. 1 is an overall configuration diagram illustrating a vital measuring device according to a first embodiment, FIG. 2 is a hardware configuration diagram of an infrared sensor 1 of the vital measuring device, and FIG. 3 is a hardware configuration diagram of a radar sensor 2 of the vital measuring device. As will be described later, in order for the radar sensor 2 to perform beam forming in a two-dimensional orientation of a measurement target detected by the infrared sensor 1, the infrared sensor 1 and the radar sensor 2 are temporally synchronized with each other and arranged at a short distance from each other. A synchronization signal for achieving temporal synchronization is supplied from a synchronization signal generation unit (not illustrated) to the infrared sensor 1 and the radar sensor 2.

(Vital Measuring Device)

The vital measuring device shown in FIG. 1 includes the infrared sensor 1, the radar sensor 2 and a final vital output unit 3.

(Infrared Sensor)

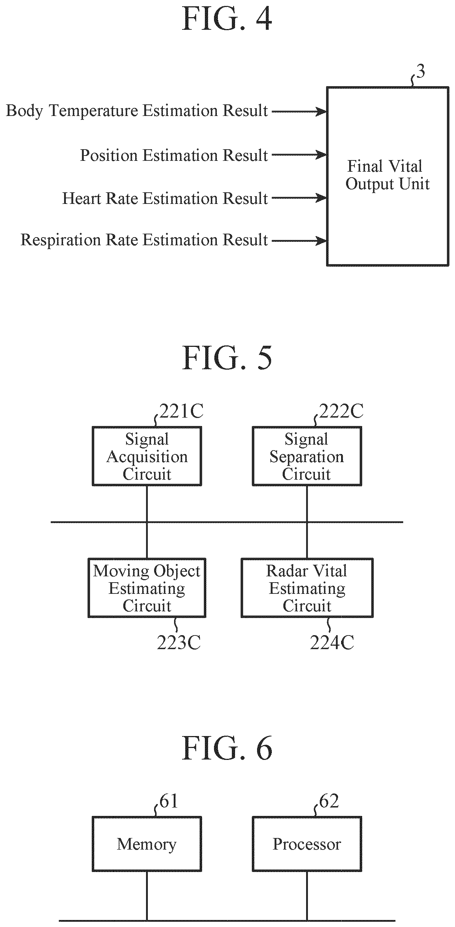

The infrared sensor 1 illustrated in FIG. 2 includes a light receiving unit 11, a biological recognition unit 12, and an infrared vital estimating unit 13. The light receiving unit 11 includes a plurality of light receiving elements arranged two-dimensionally, receives infrared light emitted from a target object, photoelectrically converts a received signal, and outputs the photoelectrically converted signal to the biological recognition unit 12 as an infrared image signal. The biological recognition unit 12 performs biological recognition such as human recognition or animal recognition by performing image recognition processing using machine learning or the like on the infrared image signal, and outputs a recognition result to the infrared vital estimating unit 13 as a biological recognition result. By using the biological recognition result, the infrared vital estimating unit 13 estimates a body temperature of a detected living body and estimates a two-dimensional orientation in which the living body is present. The biological recognition unit 12 and the infrared vital estimating unit 13 are implemented by, for example, a processor 62 and a memory 61 as illustrated in FIG. 6, similarly to a radar vital measuring unit 22 to be described later. The memory 61 stores a program and a learned model for implementing processing performed by the biological recognition unit 12 and the infrared vital estimating unit 13. The learned model is a model in which a relationship between an infrared image and a living body is learned. The processor 62 reads and executes the program stored in the memory 61, thereby implementing processing performed by the biological recognition unit 12 and the infrared vital estimating unit 13.

(Radar Sensor)

The radar sensor 2 illustrated in FIG. 3 includes a transmission and reception unit 21 and the radar vital measuring unit 22.

(Transmission and Reception Unit)

The transmission and reception unit 21 includes N antennas 211-1 to 211-N, a signal transmission unit 212, N circulators 213-1 to 213-N, and N signal reception units 214-1 to 214-N. N is an integer equal to or more than two. Each of the antennas is a transmission and reception antenna. The radar sensor 2 illustrated in FIG. 3 includes N antennas in order to increase resolution of a reception signal by multiple-input multiple-output (MIMO). Each of the antennas also serves as a transmission antenna and a reception antenna. However, this is merely an example, and the radar sensor 2 may separately include a transmission antenna and a reception antenna.

When a transmission wave is emitted from the radar sensor 2, one antenna that emits the transmission wave is selected from the antennas 211-1 to 211-N. A transmission order of the transmission waves in the antennas is determined. For example, the transmission order is determined in the order of the antenna 211-1, an antenna 211-2, ..., and an antenna 211-n. This is merely an example, and the transmission order may be determined in the order of the antenna 211-n, ..., the antenna 211-2, and the antenna 211-1, for example.

(Antenna)

The antenna 211-n (n = 1, ..., N) radiates a transmission wave related to a transmission signal output from a circulator 213-n to a space where a target object is present. The transmission wave radiated from the antenna 211-n is reflected by the target object. The target object includes not only a person to be measured present in the space but also a stationary object such as a wall of a room forming the space or a desk present in the space.

Each of the antennas 211-1 to 211-N receives a reflected wave from the target object, and outputs a reception signal of the reflected wave to the circulator.

(Signal Transmission Unit)

The signal transmission unit 212 includes a signal generator 212a and an output destination selecting unit 212b. The signal transmission unit 212 sequentially selects one antenna 211-n that emits a transmission wave from among the N antennas. In order to radiate the transmission wave from the selected antenna 211-n to the space where the person to be measured is present, the signal transmission unit 212 outputs a transmission signal to the circulator 213-n connected to the selected antenna 211-n.

(Signal Generator)

The signal generator 212a generates, for example, a transmission signal whose frequency changes with the lapse of time or a pulse transmission signal. Examples of the transmission signal whose frequency changes with the lapse of time include an up-chirp signal and a down-chirp signal. The signal generator 212a outputs the transmission signal to the output destination selecting unit 212b.

(Output Destination Selecting Unit)

The output destination selecting unit 212b outputs the transmission signal generated by the signal generator 212a to the circulator 213-n that is connected to the antenna 211-n in the next order of emitting the transmission wave among the N circulators 213.

(Circulator)

The circulator 213-n (n = 1, ..., N) outputs the transmission signal output from the output destination selecting unit 212b to the antenna 211-n. In addition, the circulator 213-n outputs the reception signal output from the antenna 211-n to a signal reception unit 214-n.

(Signal Reception Unit)

The signal reception unit 214-n (n = 1, ..., N) performs reception processing on the reception signal output from the circulator 213-n. Examples of the reception processing include processing of down-converting a frequency of the reception signal and processing of converting the reception signal after frequency conversion from an analog signal to a digital signal.
The signal reception unit 214-n outputs reception data $S(t, g, h, q, c)$, which is a digital signal, to the radar vital measuring unit 22. t represents reception time of the reflected wave by the antenna 211-n. g is a variable for identifying the antenna 211-n that has radiated the transmission wave, and g = 1, ..., N. h is a variable for identifying the antenna 211-n that has received the reflected wave, and h = 1, ..., N. q is a variable that identifies a hit number in a certain transmission cycle, and q = 1, ..., Q. Q is an integer equal to or more than two. c is a variable for identifying a transmission cycle of the transmission wave by the antennas 211-1 to 211-N, and c = 1, ..., C. C is an integer equal to or more than two.

(Radar Vital Measuring Unit)

The radar vital measuring unit 22 includes a signal acquisition unit 221, a signal separation unit 222, a moving object estimating unit 223, and a radar vital estimating unit 224.

(Signal Acquisition Unit)

The signal acquisition unit 221 acquires the reception data $S(t, g, h, q, c)$ as the reception signal of the reflected wave from each of the signal reception units 214-1 to 214-N, and outputs the reception data $S(t, g, h, q, c)$ to the signal separation unit 222.

(Signal Separation Unit)

The signal separation unit 222 calculates stationary object reception data $Ssta(t, g, h, c)$ and moving object reception data $Smov(t, g, h, q, c)$ using the reception data $S(t, g, h, q, c)$, outputs the calculated stationary object reception data $Ssta(t, g, h, c)$ to the radar vital estimating unit 224, and outputs the calculated moving object reception data $Smov(t, g, h, q, c)$ to the moving object estimating unit 223.

(Moving Object Estimating Unit)

The moving object estimating unit 223 acquires the moving object reception data $Smov(t, g, h, q, c)$ from the signal separation unit 222 and estimates a two-dimensional orientation of the moving object. The moving object estimating unit 223 outputs the estimated two-dimensional orientation of the moving object to the radar vital estimating unit 224.

(Radar Vital Estimating Unit)

The radar vital estimating unit 224 includes a stationary determination unit 2241, a Fourier transform unit 2242, a digital beam forming unit 2243, a presence position specifying unit 2244, a respiration rate estimating unit 2245, and a heart rate estimating unit 2246. The radar vital estimating unit 224 estimates a position, a respiration rate, and a heart rate of a living body from the stationary object reception data Ssta(t, g, h, c) on the basis of a detection result of the moving object detected by the moving object estimating unit 223.

(Stationary Determination Unit)

The stationary determination unit 2241 acquires the two-dimensional orientation of the moving object from the moving object estimating unit 223 and an estimation result of the two-dimensional orientation of the living body from the infrared vital estimating unit 13, respectively. It is determined whether the living body recognized by the infrared vital estimating unit 13 is stationary, and when it is determined as being stationary, a stationary determination flag is output to the Fourier transform unit 2242.

(Fourier Transform Unit)

When the stationary determination flag is acquired from the stationary determination unit 2241, the Fourier transform unit 2242 performs Fourier transform on the stationary object reception data Ssta(t, g, h, c) input from the signal separation unit 222 in a time direction. Examples of the Fourier transform include fast Fourier transform (FFT) and discrete Fourier transform (DFT). The Fourier transform unit 2242 outputs each Fourier-transformed signal Ssta(r, g, h, c) to the digital forming unit. r is a variable identifying a range bin from the vital measuring device. r = 1, ..., R. R is an integer equal to or more than two.

(Digital Beam Forming Unit)

The digital beam forming unit 2243 acquires the Fourier-transformed signal Ssta(r, g, h, c) from the Fourier transform unit 2242 and the estimation result of the two-dimensional orientation of the living body from the infrared vital estimating unit 13, respectively. The digital beam forming unit 2243 calculates a beam forming signal BFsta(r, c) by performing digital beam forming on the two-dimensional orientation of the living body obtained from the infrared vital estimating unit 13, and outputs the beam forming signal to the presence position specifying unit 2244.

(Presence Position Specifying Unit)

The presence position specifying unit 2244 acquires the beam forming signal BFsta(r, c). The presence position specifying unit 2244 estimates a vital feature amount for each range bin r on the basis of the beam forming signal BFsta(r, c), and calculates a biological slant range Rbio on the basis of magnitude of the vital feature amount. A biological signal B(c) in the biological slant range Rbio is output to the respiration rate estimating unit 2245 and the heart rate estimating unit 2246.

(Respiration Rate Estimating Unit)

The respiration rate estimating unit 2245 acquires the biological signal B(c) from the presence position specifying unit 2244. A respiration rate RR of the living body is estimated on the basis of the biological signal B(c).

(Heart Rate Estimating Unit)

The heart rate estimating unit 2246 acquires the biological signal B(c) from the presence position specifying unit 2244. A heart rate HR of the living body is estimated on the basis of the biological signal B(c).

In FIG. 3, it is assumed that each of the signal acquisition unit 221, the signal separation unit 222, the moving object estimating unit 223, and the radar vital estimating unit 224, which are components of the radar vital measuring unit 22, is implemented by dedicated hardware as illustrated in FIG. 5. That is, it is assumed that the radar vital measuring unit 22 is implemented by a signal acquisition circuit 221C, a signal separation circuit 222C, a moving object estimating circuit 223C, and a radar vital estimating circuit 224C. Each of the signal acquisition circuit 221C, the signal separation circuit 222C, the moving object estimating circuit 223C, and the radar vital estimating circuit 224C corresponds to, for example, a single circuit, a composite circuit, a programmed processor, a parallel programmed processor, an application specific integrated circuit (ASIC), a field-programmable gate array (FPGA), or a combination thereof.

(Final Vital Output Unit)

The final vital output unit 3 illustrated in FIG. 4 acquires a body temperature estimation result of the living body from the infrared sensor 1, acquires a position estimation result, a respiration rate estimation result, and a heart rate estimation result of the living body from the radar sensor 2, and outputs them to the outside. The final vital output unit 3 may be implemented by a dedicated processing circuit, similarly to the radar vital measuring unit 22. Further, as will be described below according to the radar vital measuring unit 22, it may be implemented by, for example, the processor 62 and the memory 61 as illustrated in FIG. 6. The memory 61 stores a program for implementing processing performed by the final vital output unit 3, and the processor 62 reads and executes the program stored in the memory 61, thereby implementing the processing by the final vital output unit 3.

The components of the radar vital measuring unit 22 are not limited to those implemented by dedicated hardware, and the radar vital measuring unit 22 may be implemented by software, firmware, or a combination of software and firmware.

The software or firmware is stored as a program in a memory of a computer. The computer means hardware that executes a program, and corresponds to, for example, a central processing unit (CPU), a central processing device, a processing device, an arithmetic device, a microprocessor, a microcomputer, a processor, or a digital signal processor (DSP).

FIG. 6 is a hardware configuration diagram of a computer in a case where the radar vital measuring unit 22 is implemented by software, firmware, or the like.

In a case where the radar vital measuring unit 22 is implemented by software, firmware, or the like, a program for causing the computer to execute each processing procedure performed in the signal acquisition unit 221, the signal separation unit 222, the moving object estimating unit 223, and the radar vital estimating unit 224 is stored in the memory. Then, the processor of the computer executes the program stored in the memory.

Further, FIG. 5 shows an example in which each of the components of the radar vital measuring unit 22 is implemented by dedicated hardware, and FIG. 6 shows an example in which the radar vital measuring unit 22 is implemented by software, firmware, or the like. However, this is merely an example, and some components in the radar vital measuring unit 22 may be implemented by the dedicated hardware, and the remaining components may be implemented by software, firmware, or the like.

<Operation>

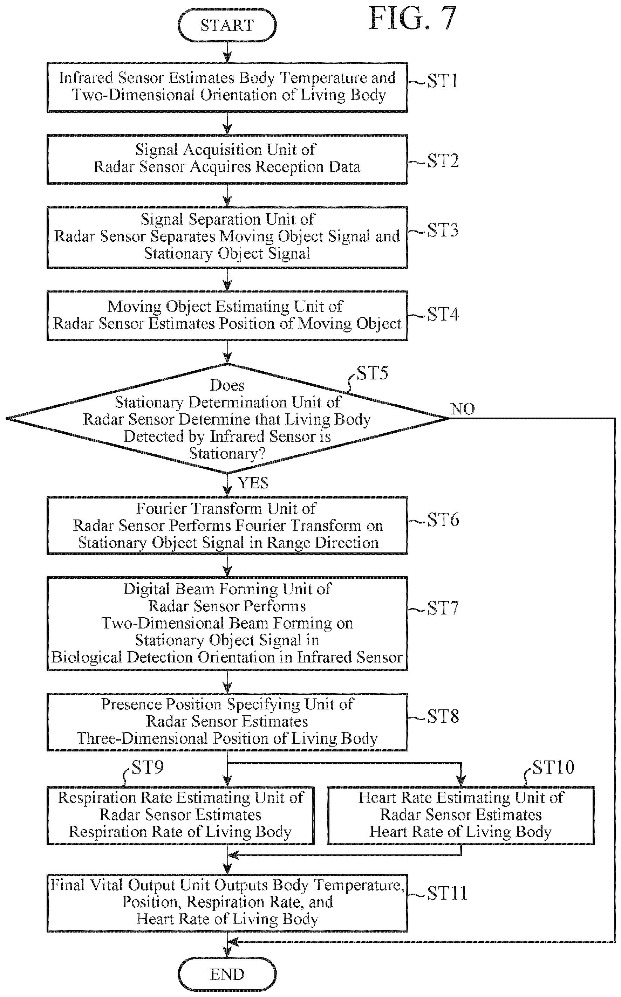

Next, an operation of the vital measuring device shown in FIG. 1 will be described with reference to FIG. 7. FIG. 7 is a flowchart illustrating a vital measuring method which is a processing procedure performed by the vital measuring device.

In step ST1 of FIG. 7, the infrared sensor 1 estimates a body temperature and a two-dimensional orientation of a living body. More specifically, the light receiving unit 11 of the infrared sensor 1 receives infrared light emitted from an object by the plurality of two-dimensionally arranged light receiving elements, photoelectrically converts a received signal, and outputs the photoelectrically converted signal to the biological recognition unit 12 as an infrared image signal.

The biological recognition unit 12 of the infrared sensor 1 specifies presence and a two-dimensional position of the living body in an infrared image by image recognition processing using machine learning or the like on the infrared image signal acquired from the light receiving unit 11. The living body includes a person or an animal.

The infrared vital estimating unit 13 of the infrared sensor 1 estimates a two-dimensional orientation of the living body as viewed from the vital measuring device and a body temperature of the living body on the basis of a biological recognition result, and outputs the estimated result.

The signal generator 212a of the signal transmission unit 212 of the radar sensor 2 generates an up-chirp signal or a down-chirp signal as a transmission signal in accordance with, for example, a frequency modulated continuous wave (FMCW) method or a fast-chirp modulation (FCM) method.

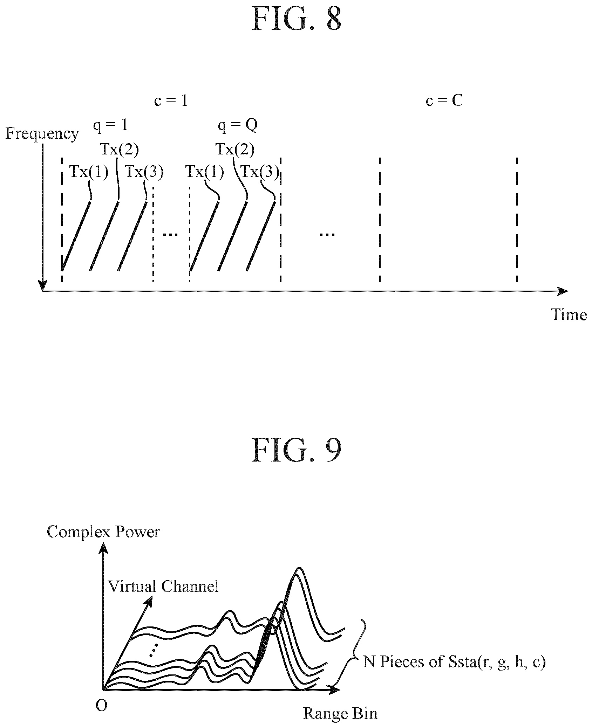

In the radar sensor 2 illustrated in FIG. 3, the signal generator 212a repeats radio wave transmission by the number of hits 1 to Q in each transmission cycle c ($c = 1, ..., C$), as illustrated in FIG. 8. Furthermore, within each hit, an up-chirp signal Tx(n) is repeatedly generated by the number of antennas 211-1 to 211-N. Therefore, the signal generator 212a generates $N \times Q \times C$ up-chirp signals Tx(n) in total. In an example of FIG. 8, N = 3. The signal generator 212a up-converts a frequency of the generated up-chirp signal Tx(n) ($n = 1, ..., N$) into a signal in a high frequency band, and outputs the up-converted signal as a transmission signal Tx'(n) to the output destination selecting unit 212b. The high frequency band is, for example, a millimeter wave band of about 30 to 300 GHz.

The output destination selecting unit 212b acquires the transmission signal Tx'(n) ($n = 1, ..., N$) from the signal generator 212a in each transmission cycle c ($c = 1, ..., C$) and each hit q ($q = 1, ..., Q$). The output destination selecting unit 212b outputs the transmission signal Tx'(n) to the circulator 213-n that is connected to the antenna 211-n in the next order of radiating the transmission wave among the N circulators 213-1 to 213-N. For example, the output destination selecting unit 212b outputs a transmission signal Tx'(1) to the circulator 213-1 when the antenna 211-n in the next order of radiating the transmission wave is the antenna 211-1, and outputs a transmission signal Tx'(2) to the circulator 213-2 when the antenna 211-n in the next order of radiating the transmission wave is the antenna 211-2. In addition, the output destination selecting unit 212b outputs a transmission signal Tx'(N) to the circulator 213-N, for example, when the antenna 211-n in the next order of radiating the transmission wave is the antenna 211-N.

Upon receiving the transmission signal Tx'(n) from the output destination selecting unit 212b, the circulator 213-n (n = 1, ..., N) outputs the transmission signal Tx'(n) to the antenna 211-n.

When receiving the transmission signal Tx'(n) from the circulator 213-n, the antenna 211-n (n = 1, ..., N) radiates a transmission wave, which is a radio wave related to the transmission signal Tx'(n), to a space where a target object is present. The transmission wave radiated from the antenna 211-n is reflected by the target object. That is, the transmission wave radiated from the antenna 211-n is reflected not only by a person to be measured but also by a wall or the like forming the space. The reflected wave from the person to be measured may be further reflected by a wall or the like of a room where the person to be measured is present.

In each transmission cycle c (c = 1, ..., C) and each hit q (q = 1, ..., Q), since one antenna among the N antennas 211-1 to 211-N sequentially radiates the transmission wave into the space N times, each of the antennas 211-1 to 211-N receives the reflected wave N × Q times in each transmission cycle c.

The antenna 211-n (n = 1, ..., N) outputs a reception signal including the reflected wave from the target object to the circulator 213-n. The circulator 213-n (n = 1, ..., N) outputs the reception signal output from the antenna 211-n to the signal reception unit 214-n.

The signal reception unit 214-n (n = 1, ..., N) performs reception processing on each of the N × Q reception signals output from the circulator 213-n in each transmission cycle c (c = 1, ..., C). The signal reception unit 214-n performs, for example, processing of down-converting a frequency of the reception signal to a frequency in an intermediate frequency band and processing of converting the reception signal after frequency conversion from an analog signal to a digital signal as the reception processing for the reception signal. In each transmission cycle c, the signal reception unit 214-n outputs each reception data S(t, g, h, q, c), which is N × Q digital signals, to the radar vital measuring unit 22. As a result, in each transmission cycle c (c = 1, ..., C), a total of N × N × Q (= G × H × Q) pieces of reception data S(t, g, h, q, c) are provided from the signal reception units 214-1 to 214-N to the radar vital measuring unit 22.

In step ST2, the signal acquisition unit 221 of the radar vital measuring unit 22 acquires N × N × Q pieces of reception data S(t, g, h, q, c) from the signal reception units 214-1 to 214-N in each transmission cycle c (c = 1, ..., C). The signal acquisition unit 221 outputs N × N × Q pieces of reception data S(t, g, h, q, c) to the signal separation unit 222 in each transmission cycle c.

In step ST3, the signal separation unit 222 separates a moving object signal and a stationary object signal. First, the signal separation unit 222 acquires N × N × Q pieces of reception data S(t, g, h, q, c) from the signal acquisition unit 221 in each transmission cycle c (c = 1, ..., C). Next, the signal separation unit 222 outputs stationary object reception data Ssta(t, g, h, c) obtained by averaging all the hits q (q = 1, ..., Q) with respect to the reception data S(t, g, h, q, c) to the radar vital estimating unit 224. Further, the signal separation unit 222 outputs moving object reception data Smov(t, g, h, q, c) obtained by subtracting the stationary object reception data Ssta(t, g, h, c) from the reception data S(t, g, h, q, c) to the moving object estimating unit 223.

In step ST4, the moving object estimating unit 223 estimates a position of a moving object. First, the moving object estimating unit 223 acquires N × N × Q pieces of moving object reception data Smov(t, g, h, q, c) from the signal separation unit 222 in each transmission cycle c (c = 1, ..., C). The moving object estimating unit 223 estimates a range from the radar sensor 2 to the moving object and a velocity of the moving object by performing range Doppler processing on the moving object present in the space on the basis of the acquired N × N × Q pieces of moving object reception data Smov(t, g, h, q, c). Since the range Doppler processing itself is a known technique, detailed description thereof will be omitted. For example, when the number of moving objects present in the space is one, a range to one moving object and a velocity of the moving object are estimated. For example, when a first moving object and a second moving object are present in the space as two moving objects, a range to the first moving object and a velocity of the first moving object, and a range to the second moving object and a velocity of the second moving object are estimated. The moving object estimating unit 223 detects a two-dimensional orientation in which the moving object is present by performing angle measuring processing on the basis of estimation results of the range to the moving object and the velocity of the moving object. The two-dimensional orientation has an azimuth direction in which the moving object is viewed from the vital measuring device and an elevation direction in which the moving object is viewed from the vital measuring device.

In step ST5, the stationary determination unit 2241 determines whether or not the living body detected by the infrared sensor 1 is stationary. In order to make this determination, the stationary determination unit 2241 acquires a two-dimensional orientation of the moving object from the moving object estimating unit 223, and acquires an estimation result of the two-dimensional orientation of the living body from the infrared vital estimating unit 13, respectively. When an error between the two-dimensional orientation where the moving object is present obtained from the moving object estimating unit 223 and the two-dimensional orientation of the living body obtained from the infrared vital estimating unit 13 is less than a predetermined threshold, the stationary determination unit 2241 determines that the living body detected by the infrared sensor 1 is moving. On the other hand, when the error is equal to or larger than the predetermined threshold, the stationary determination unit 2241 determines that the living body detected by the infrared sensor 1 is stationary and outputs a stationary determination flag to the Fourier transform unit 2242.

When the stationary determination flag is acquired from the stationary determination unit 2241 (Yes in step ST5), the processing proceeds to step ST6. In step ST6, the Fourier transform unit 2242 performs Fourier transform on each of the $N \times N \times Q$ pieces of stationary object reception data Ssta(t, g, h, c) in a time direction in each transmission cycle c. In each transmission cycle c, each of the N pieces of stationary object reception data Ssta(t, g, h = n, c) related to each antenna 211-n is Fourier-transformed by the Fourier transform unit 2242, whereby N pieces of Fourier-transformed signals Ssta(r, g, h, c) are generated. The signals Ssta(r, g, h, c) after the Fourier transform by the Fourier transform unit 2242 are signals indicating complex power corresponding to the range bin r as illustrated in FIG. 9. FIG. 9 is an explanatory diagram illustrating N pieces of Fourier-transformed signals Ssta(r, g, h, c) in a certain transmission cycle c. In FIG. 9, the horizontal axis represents the range bin and the vertical axis represents the complex power. The Fourier transform unit 2242 outputs N pieces of Fourier-transformed signals Ssta(r, g, h, c) to the digital beam forming unit 2243 in each transmission cycle c.

In step ST7, the digital beam forming unit 2243 performs two-dimensional beam forming on the stationary object signal in a biological detection orientation in the infrared sensor 1, and calculates a beam forming signal BF(r, c). In order to perform such processing, the digital beam forming unit 2243 acquires the Fourier-transformed signal Ssta(r, g, h, c) from the Fourier transform unit 2242 and the estimation result of the two-dimensional orientation of the living body from the infrared vital estimating unit 13, respectively. The digital beam forming unit 2243 calculates the beam forming signal BF(r, c) by performing digital beam forming on the two-dimensional orientation of the living body obtained from the infrared vital estimating unit 13, and outputs the calculated beam forming signal BF(r, c) to the presence position specifying unit 2244.

More specifically, the digital beam forming unit 2243 performs, on each Fourier-transformed signal Ssta(r, g, h, c), two-dimensional orientation beam forming processing by digital beam forming (DBF) toward the two-dimensional orientation of the living body obtained from the infrared vital estimating unit 13 for each range bin r. Here, the two-dimensional orientation includes the azimuth direction and the elevation direction. However, this is merely an example, and in the digital beam forming, the two-dimensional orientation beam forming processing may be performed using another method such as a Capon method. Note that the two-dimensional orientation beam forming processing itself is a known technique, and thus a detailed description thereof will be omitted. In each transmission cycle c, the digital beam forming unit 2243 outputs a beam forming signal BF(r, c) corresponding to each range bin r to the presence position specifying unit 2244.

Since the two-dimensional orientation of the living body has been estimated by the processing so far, in step ST8, the presence position specifying unit 2244 estimates a remaining biological slant range necessary for estimating a three-dimensional position of the living body. Hereinafter, position specifying processing by the presence position specifying unit 2244 will be specifically described.

Although a respiration signal indicating respiration of the living body and a heartbeat signal indicating heartbeat of the living body are superimposed on the reflected wave from the living body, most of the beam forming signal BF(r, c) is power of the respiration signal.

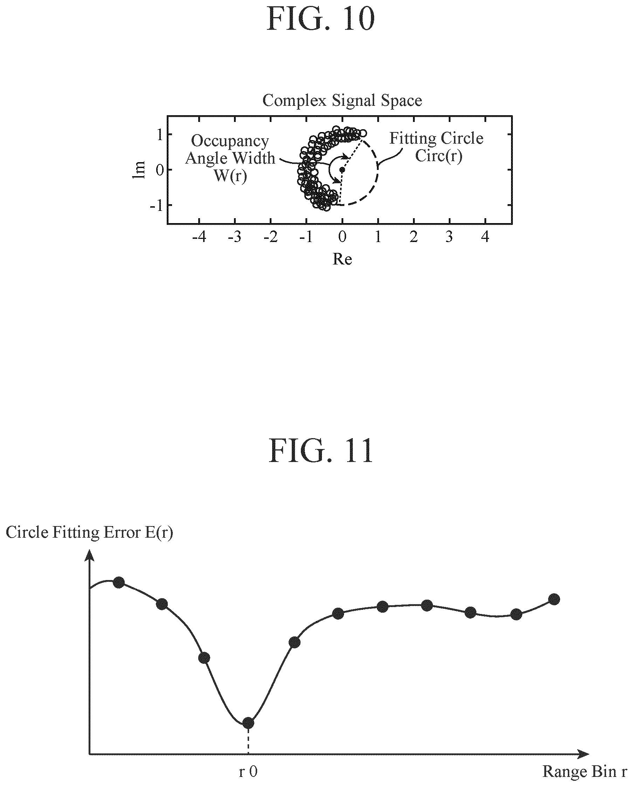

The respiration signal is a signal indicating a phase variation caused by a reciprocating motion in a chest of the living body. Therefore, the beam forming signals BF(r, c) belonging to the same range bin (slant range bin) r are time-varying signals, and exhibit the reciprocating motion on a circumference as time passes in a complex signal space as illustrated in FIG. 10. FIG. 10 is an explanatory diagram illustrating temporal changes of phases of beam forming signals BF(r, c) belonging to the same range bin r. In FIG. 10, the beam forming signals BF(r, c) exhibit the reciprocating motion on the circumference in the complex signal space. In FIG. 10, a circle indicates a beam forming signal BF(r, c) in the transmission cycles c = 1 to C, and C circles are plotted.

The presence position specifying unit 2244 calculates a fitting circle Circ(r) drawn by temporal changes of a phase of the beam forming signal BF(r, c) corresponding to each range bin r (r = 1, ..., R) on a complex number plane. Specifically, assuming that complex data indicating the beam forming signal BF(r, c) in the transmission cycle c (c = 1, ..., C) is $s_c$, the presence position specifying unit 2244 calculates the optimum fitting circle Circ(r) by calculating $\alpha$ and $\beta$ whose evaluation function J($\alpha$, $\beta$) shown in the following Formula (1) is minimized. $\alpha$ is a center of the fitting circle Circ(r), and $\beta$ is a radius of the fitting circle Circ(r). A subscript c of $s_c$ is a variable indicating the transmission cycle.

$$J(\alpha, \beta) = \sum_{c=1}^{C} (|s_c - \alpha|^2 - \beta^2)^2 \qquad (1)$$

The evaluation function J($\alpha$, $\beta$) can be expressed in a matrix form as shown in the following Formula (2) by formula deformation.

$$J(\alpha, \beta) = \sum_{c=1}^{C} (|s_c - \alpha|^2 - \beta^2)^2$$

$$= \sum_{c=1}^{C} ((s_c - \alpha)(s_c^* - \alpha^*) - \beta^2)^2$$

$$= \sum_{c=1}^{C} |s_c s_c^* - \alpha s_c^* - \alpha^* s_c + \alpha\alpha^* - \beta^2|^2$$

$$= \sum_{c=1}^{C} \left| s_c s_c^* - [s_c^* \quad s_c \quad 1] \begin{bmatrix} \alpha \\ \alpha^* \\ \beta^2 - \alpha\alpha^* \end{bmatrix} \right|^2$$

$$= \left| \begin{bmatrix} s_1 s_1^* \\ s_2 s_2^* \\ \vdots \\ s_C s_C^* \end{bmatrix} - \begin{bmatrix} s_1^* & s_1 & 1 \\ s_2^* & s_2 & 1 \\ \vdots & \vdots & \vdots \\ s_C^* & s_C & 1 \end{bmatrix} \begin{bmatrix} \alpha \\ \alpha^* \\ \beta^2 - \alpha\alpha^* \end{bmatrix} \right|^2$$

$$\triangleq |s - Hp|^2 \qquad\qquad (2)$$

In Formula (2), * is a mathematical symbol representing a complex conjugate.

The presence position specifying unit 2244 estimates a parameter vector p shown in Formula (2) using the least squares method as shown in Formula (3) below.

$$\widetilde{p} = H^\dagger s \qquad\qquad (3)$$

In Formula (3), + is a mathematical symbol representing a pseudo inverse matrix. "~" displayed above the letter p is a symbol indicating an estimation result of the parameter vector p.

Assuming that an estimation result of the parameter vector p is expressed by the following Formula (4), the presence position specifying unit 2244 can obtain the center $\alpha$ of the fitting circle Circ(r) and the radius $\beta$ of the fitting circle Circ(r) as expressed by the following Formulas (5) and (6). When the center $\alpha$ of the fitting circle Circ(r) and the radius $\beta$ of the fitting circle Circ(r) are obtained, the fitting circle Circ(r) is calculated.

$$\widetilde{p} = [p_1, p_2, p_3]^T \qquad\qquad (4)$$

$$\alpha = \frac{Re(p_1 + p_2)}{2} + j\frac{Im(p_1 - p_2)}{2} \qquad\qquad (5)$$

$$\beta = \sqrt{Re(p_3{}^2 + p_1 p_2)} \qquad\qquad (6)$$

In Formulas (5) and (6), Re($\square$) represents a real part of a complex number $\square$, and Im(o) represents an imaginary part of the complex number $\square$.

Here, the presence position specifying unit 2244 calculates the fitting circle Circ(r) using a circle fitting method called Kasa fit. However, this is merely an example, and the presence position specifying unit 2244 may calculate the fitting circle Circ(r) using a circle fitting method such as Pratt fit, Taubin fit, or Hyper fit.

Next, the presence position specifying unit 2244 calculates a fitting error E(r) which is an error of the fitting circle Circ(r) as expressed in the following Formula (7).

$$E(r) = \sqrt{\sum_{c=1}^{C} \left| \frac{BF(r,c) - \alpha(r)}{\beta(r)} - 1 \right|^2} \qquad (7)$$

In Formula (7), $\alpha(r)$ is the center of the fitting circle Circ(r), and $\beta(r)$ is the radius of the fitting circle Circ(r).

If the respiration signal indicating the respiration of the living body and the heartbeat signal indicating the heartbeat of the living body are superimposed on the reflected wave, the radius $\beta$ of the fitting circle Circ(r) becomes large and the fitting error E(r) becomes small. On the other hand, if the reflected wave is a reflected wave from a stationary object such as a wall, that is, a direct wave from the stationary object, the radius $\beta$ of the fitting circle Circ(r) becomes small and the fitting error E(r) becomes large.

The presence position specifying unit 2244 sets the calculated r fitting errors E(r) as vital feature amounts, and compares the fitting errors E(r) in a range bin r direction. That is, the presence position specifying unit 2244 compares the fitting errors E(r), which are the vital feature amounts, between the slant range bins. On the basis of a result of this comparison, the presence position specifying unit 2244 estimates a range bin r0, at which the fitting error E(r) is minimized, as the slant range to the living body. FIG. 11 is an explanatory diagram illustrating a change of the fitting error E(r) with respect to the range bin r. FIG. 11 illustrates a state in which the fitting error E(r) is minimized in the range bin r0. In FIG. 11, • indicates the fitting error E(r) at the range bins r = 1 to R, and R pieces of • are plotted.

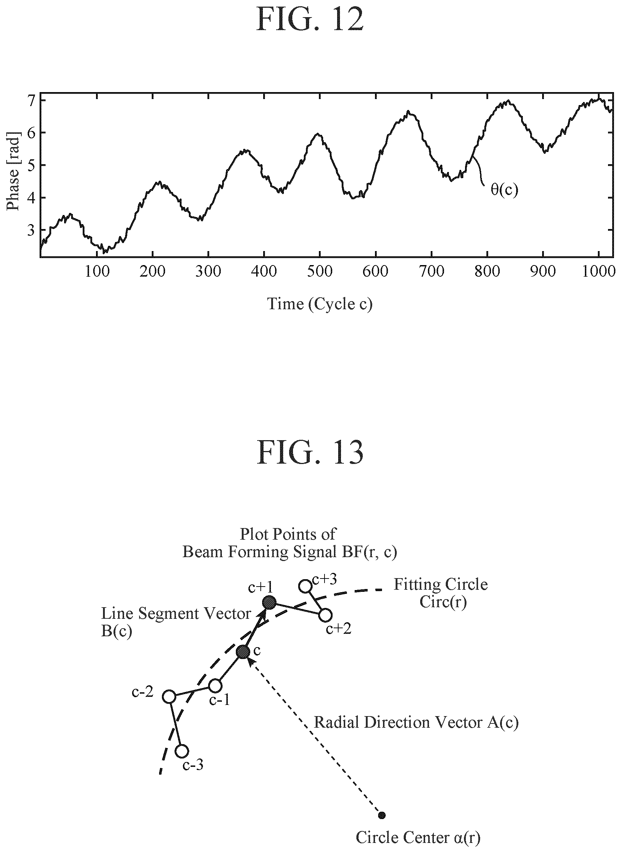

In step ST9, the respiration rate estimating unit 2245 estimates a respiration rate RR of the living body. First the respiration rate estimating unit 2245 calculates a phase change signal $\theta(c)$ as illustrated in FIG. 12 from a beam forming signal BF(r0, c) for the range bin r0 at which the living body is present in the transmission cycles c = 1 to C. The respiration rate estimating unit 2245 can use, for example, an arctangent demodulation (AD) method or a complex signal demodulation (CSD) method as a method of calculating the phase change signal $\theta(c)$ for a position where the living body is present. Next, the respiration rate estimating unit 2245 estimates the respiration rate RR of the living body by performing Fourier transform on the phase change signal $\theta(c)$. Note that a technique for estimating the respiration rate RR using the phase change signal $\theta(c)$ itself is a known technique, and thus a detailed description thereof is omitted.

In step ST10, the heart rate estimating unit 2246 similarly calculates the phase change signal $\theta(c)$ as illustrated in FIG. 12 from the beam forming signal BF(r0, c) for the range bin r0 at which the living body is present in the transmission cycles c = 1 to C. The heart rate estimating unit 2246 can also use, for example, the AD method or the CSD method as the method of calculating the phase change signal $\theta(c)$ for the position where the living body is present, but it is not necessary to use the same method as that of the respiration rate estimating unit 2245. The heart rate estimating unit 2246 estimates a heart rate HR of the living body by performing Fourier transform or wavelet transform on the phase change signal $\theta(c)$. Note that a technique for estimating the heart rate HR using the phase change signal $\theta(c)$ itself is a known technique, and thus a detailed description thereof is omitted.

In step ST11, the final vital output unit 3 acquires a body temperature estimation result of the living body from the infrared sensor 1, acquires a position estimation result, a respiration rate estimation result, and a heart rate estimation result of the living body from the radar sensor 2, and outputs them to the outside.

Second Embodiment

In a second embodiment, a configuration is the same as that of the first embodiment, but the radius $\beta(r)$ of the fitting circle Circ(r) is used as the vital feature amount in the presence position specifying unit 2244. The presence position specifying unit 2244 sets calculated R fitting circle radii $\beta(r)$ as the vital feature amounts, compares the fitting circle radii $\beta(r)$ in the range bin r direction, and estimates a range bin r0, at which the fitting circle radius $\beta(r)$ is maximized, as the slant range to the living body.

Third Embodiment

In a third embodiment, a configuration is the same as that of the first embodiment, but an occupancy angle width W(r), in which the beam forming signal BF(r, c) occupies on the fitting circle Circ(r) in the transmission cycle c = 1 to C, is used as the vital feature amount in the presence position specifying unit 2244. The presence position specifying unit 2244 sets calculated R occupancy angle widths W(r) as the vital feature amounts, compares the occupancy angle widths W(r) in the range bin r direction, and estimates a range bin r0, at which the occupancy angle width W(r) is maximized, as the slant range to the living body. FIG. 10 illustrates the occupancy angle width W(r).

Fourth Embodiment

In a fourth embodiment, a configuration is the same as that of the first embodiment, but an inner product of a radial direction vector A(c) from the circle center $\alpha$(r) of the fitting circle Circ(r) toward each signal point (time-varying signal point) of the beam forming signal BF(r, c) and a line segment vector B(c) connecting adjacent signal points of the beam forming signal BF(r, c) is calculated, and a total value I(r) of inner product values corresponding to all the beam forming signal points is set as the vital feature amount in the presence position specifying unit 2244. The total value I(r) of the inner product values is calculated by Formula (8). When a fitting error of the beam forming signal point to the fitting circle Circ(r) is small, each inner product value becomes small, and as a result, the total value I(r) of the inner product values also becomes small.

$$I(r) = \sum_{c=1}^{C-1} B(c)^T A(c) \qquad (8)$$

The presence position specifying unit 2244 sets the calculated R inner product total values I(r) as vital feature amounts, compares the inner product total values I(r) in the range bin r direction, and estimates the range bin r0 at which the inner product total value I(r) is minimum as the slant range to the living body. FIG. 13 illustrates an example of vectors used to calculate an inner product value corresponding to a certain adjacent beam forming signal point pair.

Note that the embodiments can be combined, and the embodiments can be appropriately modified or omitted.

INDUSTRIAL APPLICABILITY

The vital measuring technique of the present disclosure can be used as a vital measuring device that measures vitals of one or a small number of elderly people.

REFERENCE SIGNS LIST

1: infrared sensor, 2: radar sensor, 3: final vital output unit, 11: light receiving unit, 12: biological recognition unit, 13: infrared vital estimating unit, 21: transmission and reception unit, 22: radar vital measuring unit, 61: memory, 62: processor, 211-n: antenna, 212: signal transmission unit, 212a: signal generator, 212b: output destination selecting unit, 213-n: circulator, 214-n: signal reception unit, 221: signal acquisition unit, 221C: signal acquisition circuit, 222: signal separation unit, 222C: signal separation circuit, 223: moving object estimating unit, 223C: moving object estimating circuit, 224: radar vital estimating unit, 224C: radar vital estimating circuit, 2241: stationary determination unit, 2242: Fourier transform unit, 2243: digital beam forming unit, 2244: presence position specifying unit, 2245: respiration rate estimating unit, 2246: heart rate estimating unit

**Claims**

1. A vital measuring device comprising:

a single infrared sensor including
a light receiving unit to receive infrared light emitted from a target object and capture an infrared image,
a biological recognition unit to recognize a living body from the infrared image captured by the light receiving unit, and
an infrared vital estimating unit to estimate a body temperature and a two-dimensional orientation of the living body recognized by the biological recognition unit;
a single radar sensor temporally synchronized with the infrared sensor and disposed in proximity to the infrared sensor in such a manner as to sense the living body,
the single radar sensor including
a signal acquisition unit to acquire a reception signal of a reflected wave from an antenna for receiving the reflected wave from the target object,
a signal separation unit to separate the reception signal acquired by the signal acquisition unit into a moving object signal and a stationary object signal,
a moving object estimating unit to detect a moving object by estimating a range, a velocity, and a two-dimensional orientation of the moving object from the moving object signal, and
a radar vital estimating unit to estimate a position, a respiration rate, and a heart rate of the living body from the stationary object signal on a basis of a detection result of the moving object detected by the moving object

**EP 4 555 926 A1**

estimating unit; and

a final vital output unit to acquire the position, the respiration rate, and the heart rate of the living body estimated by the radar vital estimating unit, acquire the body temperature of the living body estimated by the infrared vital estimating unit, to output the acquired position, body temperature, respiration rate, and heart rate of the living body.

2. The vital measuring device according to claim 1, wherein
the radar vital estimating unit includes a digital beam forming unit to perform digital beam forming of the stationary object signal in the estimated two-dimensional orientation when movement of the moving object detected by the moving object estimating unit of the radar sensor is not detected in the two-dimensional orientation estimated by the infrared vital estimating unit.

3. The vital measuring device according to claim 2, wherein
the radar vital estimating unit compares vital feature amounts between slant range bins in the beam-formed stationary object signal, determines that the living body is present in a slant range bin having a maximum or minimum vital feature amount, and estimates a respiration rate and a heart rate in the slant range bin.

4. The vital measuring device according to claim 3, wherein
a time-varying signal of a signal in the slant range bins of the beam-formed stationary object signal is circular fitted on a complex number plane, and an error between the fitted circle and the time-varying signal is set as the vital feature amount.

5. The vital measuring device according to claim 3, wherein
a time-varying signal of a signal in the slant range bins of the beam-formed stationary object signal is circular fitted on a complex number plane, and a circle radius of the fitted circle is set as the vital feature amount.

6. The vital measuring device according to claim 3, wherein
a time-varying signal of a signal in the slant range bins of the beam-formed stationary object signal is circular fitted on a complex number plane, and an occupancy angle width of the time-varying signal viewed from a center of the fitted circle is set as the vital feature amount.

7. The vital measuring device according to claim 3, wherein
a time-varying signal of a signal in the slant range bins of the beam-formed stationary object signal is circular fitted on a complex number plane, an inner product of a radial direction vector from a center of the fitted circle toward a point of the time-varying signal and a line segment vector connecting the adjacent points of the time-varying signal is calculated, and a total value of inner product values corresponding to all the points of the time-varying signal is set as the vital feature amount.

8. A vital measuring method performed by a vital measuring device including a single infrared sensor, a single radar sensor having a signal acquisition unit, a signal separation unit, a moving object estimating unit, and a radar vital estimating unit, and a final vital output unit,
the vital measuring method comprising:

receiving, by the infrared sensor, infrared light emitted from a target object to capture an infrared image, recognize a living body from the captured infrared image, and estimate a body temperature and a two-dimensional orientation of the recognized living body;
acquiring, by the signal acquisition unit, a reception signal of a reflected wave from an antenna for receiving the reflected wave from the target object;
separating, by the signal separation unit, the acquired reception signal into a moving object signal and a stationary object signal;
detecting, by the moving object estimating unit, a moving object by estimating a range, a velocity, and a two-dimensional orientation of the moving object from the moving object signal;
estimating, by the radar vital estimating unit, a position, a respiration rate, and a heart rate of the living body from the stationary object signal on a basis of a detection result of the detected moving object; and
acquiring, by the final vital output unit, the estimated position, respiration rate, and heart rate of the living body and the estimated body temperature of the living body, to output the acquired position, body temperature, respiration rate, and heart rate of the living body.

14

# FIG. 1

# FIG. 2

# FIG. 3

Estimation Result of
Two-Dimensional Orientation of Living Body

Radar Sensor 2

21

212 Signal Transmission Unit

212b Output Destination Selecting Unit

212a Signal Generator

211-1

213-1

214-1 Signal Reception Unit

211-2

213-2

214-2 Signal Reception Unit

211-N

213-N

214-N Signal Reception Unit

22 Radar Vital Measuring Unit

221 Signal Acquisition Unit

222 Signal Separation Unit

223 Moving Object Estimating Unit

224

2242 Fourier Transform Unit

2241 Stationary Determination Unit

2243 Digital Beam Forming Unit

2244 Presence Position Specifying Unit

2245 Respiration Rate Estimating Unit

2246 Heart Rate Estimating Unit

Radar Vital Estimating Unit

Position Estimation Result

Heart Rate Estimation Result

Respiration Rate Estimation Result

EP 4 555 926 A1

16

# FIG. 4

Body Temperature Estimation Result ⟶

Position Estimation Result ⟶

Heart Rate Estimation Result ⟶

Respiration Rate Estimation Result ⟶

3

Final Vital
Output Unit

# FIG. 5

221C

Signal
Acquisition
Circuit

222C

Signal
Separation
Circuit

Moving Object
Estimating
Circuit

Radar Vital
Estimating
Circuit

223C

224C

# FIG. 6

61

Memory

62

Processor

17

# FIG. 7

```
          ( START )

Infrared Sensor Estimates Body Temperature and      ~ST1
   Two-Dimensional Orientation of Living Body

        Signal Acquisition Unit of                  ~ST2
   Radar Sensor Acquires Reception Data

        Signal Separation Unit of                    ~ST3
Radar Sensor Separates Moving Object Signal and
        Stationary Object Signal

    Moving Object Estimating Unit of                 ~ST4
Radar Sensor Estimates Position of Moving Object

                                          ST5
              Does
     Stationary Determination Unit of
   Radar Sensor Determine that Living Body        NO
   Detected by Infrared Sensor is
              Stationary?
                  │ YES

        Fourier Transform Unit of                    ~ST6
Radar Sensor Performs Fourier Transform on
   Stationary Object Signal in Range Direction

       Digital Beam Forming Unit of                  ~ST7
          Radar Sensor Performs
     Two-Dimensional Beam Forming on
          Stationary Object Signal in
Biological Detection Orientation in Infrared Sensor

    Presence Position Specifying Unit of             ~ST8
          Radar Sensor Estimates
   Three-Dimensional Position of Living Body

  ST9                                      ST10
Respiration Rate Estimating Unit of     Heart Rate Estimating Unit of
     Radar Sensor Estimates                Radar Sensor Estimates
Respiration Rate of Living Body          Heart Rate of Living Body

Final Vital Output Unit Outputs Body Temperature,   ~ST11
      Position, Respiration Rate, and
         Heart Rate of Living Body

            ( END )
```

# FIG. 8

# FIG. 9

## FIG. 10

Complex Signal Space

Occupancy Angle Width W(r)

Fitting Circle Circ(r)

## FIG. 11

Circle Fitting Error E(r)

r 0

Range Bin r

# FIG. 12

# FIG. 13

**EP 4 555 926 A1**

## INTERNATIONAL SEARCH REPORT

| International application No. |
|---|
| **PCT/JP2022/031638** |

**A. CLASSIFICATION OF SUBJECT MATTER**

*A61B 5/11*(2006.01)i; *A61B 5/01*(2006.01)i; *A61B 5/113*(2006.01)i
FI: A61B5/11 110; A61B5/113; A61B5/01 350

According to International Patent Classification (IPC) or to both national classification and IPC

**B. FIELDS SEARCHED**

Minimum documentation searched (classification system followed by classification symbols)

A61B5/11; A61B5/01; A61B5/113

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | WO 2022/072838 A1 (DAWNLIGHT TECHNOLOGIES INC.) 07 April 2022 (2022-04-07) paragraphs [0041]-[0045], [0073] | 1, 8 |
| A | paragraphs [0041]-[0045], [0073] | 2-7 |
| Y | KR 10-2021-0006225 A (SK TELECOM CO., LTD.) 18 January 2021 (2021-01-18) paragraphs [0016]-[0084], fig. 8, 9 | 1, 8 |
| A | paragraphs [0016]-[0084], fig. 8, 9 | 2-7 |
| A | WO 2022/075467 A1 (SAKURATECH CO., LTD.) 14 April 2022 (2022-04-14) paragraphs [0043]-[0094], [0121]-[0129], [0148]-[0155] | 1-8 |
| A | JP 2019-50859 A (JAPAN RADIO CO., LTD.) 04 April 2019 (2019-04-04) paragraphs [0001]-[0075] | 3-7 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **14 September 2022** | **27 September 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/031638**

| Patent document cited in search report | Publication date (day/month/year) | Patent family member(s) | Publication date (day/month/year) |
|---|---|---|---|
| WO 2022/072838 A1 | 07 April 2022 | (Family: none) | |
| KR 10-2021-0006225 A | 18 January 2021 | (Family: none) | |
| WO 2022/075467 A1 | 14 April 2022 | (Family: none) | |
| JP 2019-50859 A | 04 April 2019 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DE-MING CHIAN** ; **CHAO-KAI WEN** ; **CHANG-JEN WANG** ; **MING-HUAN HSU** ; **FU-KANG WANG**. Vital Signs Identification System with Doppler Radars and Thermal Camera. *IEEE Transactions on Biomedical Circuits and Systems*, February 2022, vol. 16 (1), 153-167